# EUROPEAN PATENT APPLICATION

(11) **EP 0 628 287 A2**
(43) Date of publication of application: **14.12.1994**
(21) Application number: 94106489.1
(22) Date of filing: 26.04.1994
(51) Int. Cl.: A61B 17/16

(54) **Magnetic device for determining the location and orientation of a hole through an intramedullary nail**

(30) Priority: 20.05.1993 IT MI931037
(71) Applicant: Valsecchi, Maurizio Luigi, I-20080 Casarile (MI) (IT)
(72) Inventor: Valsecchi, Maurizio Luigi, I-20080 Casarile (MI) (IT)
(74) Representative: Trupiano, Roberto

(57) **Abstract**

Magnetic device for the individuation of the location and direction of the bone perforation axis, particularly of the distal hole (3) of universal nails (1) utilized in the reduction of bone fractures, constituted by a cylindrical permanent magnet (11) inserted, through a guided support (13), into the cavity of the nail (1), until its axis is positioned coaxially to that of the distal hole (3), by a translable and rotary distal centering device (15) in touch with the bone surface (10) near said distal hole (3), and by a flat element (16) coaxial relatively to said centering device (15), which element is provided with three (or more) probes (17,17a,17b) which take the magnetic intensity of the magnet and are arranged at 120° to one another on a circumference, said three probes (17,17a,17b) being connected to a viewercomputer programmed to indicate the values taken by said probes and to calculate the mean, so as to allow said centering device (15) in touch with the bone, by translating and rotating about itself, to individuate the location and direction of the axis of the magnet (11) and therefore that of the distal hole (3) of the nail.

## Description

This invention relates to a magnetic device for the individuation of the location and direction of the axis of holes to be made in bones in case of reduction of bone fractures by driving in of protheses generally called universal nails or intramedulllary nails.

As is known, the intramedullary nail utilized in the reduction of bone fractures is substantially an elongated and slightly arched hollow metal body, provided near the opposing ends with at least two transversal through holes, one of which is called a "proximal" hole, being rather near the operator who performs the driving in, and the other one is called a "distal" hole; besides, the end near the proximal hole is provided with a conical threading suitable to house a grip for driving in the nail.

The distal hole is necessary to allow the correct and stable fastening of the nail to the bone section or fragment to be coupled to the bone fragment already fastened to said nail through a screw or a bolt inserted into the proximal hole.

In practice, while it is rather easy to individuate, once the nail is inserted, the location of the proximal hole, and realizing therefore the corresponding hole through the bone in order to allow the nailing with a screw or the like, it is very difficult to individuate exactly the location and direction of the distal hole, because of the arched form and the length of the nail.

The present art utilizes, for driving in the nail, an angled or arched grip, mounted on the proximal end of the nail and axially provided with a bolt on which axial hits are directed by means of a ram, while for the individuation of the location and direction of the distal hole, unhealthy X-ray devices are usually utilized, which consist of a so-called "distal centering device", incorporating a laying viewer and a generator of directional waves (known as a Trocar), which is pushed, throuh a cut made in the muscle enclosing the fracturated bone, until the surface of said bone is reached in a location approximately near the one of the distal hole of the intramedullary nail inserted.

By shifting the centering device on the bone surface, the location of the distal round hole is indicated by the directional radiation emitted by the Trocar, while the direction of its axis is indicated through a point-centering device associated to the distal centering device upon removal of the directional trocar. Having individuated the distal hole, a hole is then made axially through the bone, in coincidence with the axis of the distal hole.

These known techniques for the individuation of the location and direction of the distal hole involve in practice the utilization of complicated apparatuses and especially of X-rays, that, as is known, are harmful for men.

Besides, the exactness obtainable as concern the alignment of the axis of the distal hole with that of the hole made in the bone is not always fully satisfactory, as the exactness of the location and direction of the hole, seen through the distal centering device and the subsequent point-centering device, depends on the skill and experience of the operator.

Object of this invention is therefore to provide a simple and highly efficient device for the individuation of the location and direction of the distal hole, capable of obviating the limitations of the known devices, and especially such as to avoid the utilization of harmful radiations.

A further object of this invention is to provide a device of the above specified type, so designed as to be easily utilizable, highly reliable and of limited overall dimensions.

These and still further objects which shall be more clearly stressed by the following description are reached by a magnetic activity device for the individuation of the correct location and direction of the axis of a hole, particularly of the distal hole, in order to allow to make a corresponding hole in the bones for anchoring hollow universal nails provided with a proximal hole, a distal hole and a conical threading on the proximal end, which device is constituted, according to this invention, by a permanent cylindrical magnet, transversally anchored to an end of a bar-shaped element guidedly inserted into the existing cavity of the universal nail, until the geometrical axis of said magnet is stably located coaxially to that of said distal hole; by a bar-shaped cone-pointed element with the function of distal centering device which is caused to translate until it comes in touch with the bone surface in the region of the distal hole to be individuated; by a flat element, anchored coaxially and transversally to said distal centering device and supporting three probes which take the intensity of the magnetic field emitted by said magnet and arranged at 120° to one another on a circumference, said three probes being connected to a viewer-computer programmed to elaborate and show the values of the measurements of the three probes and to supply the mean of said three values, so as to allow, by shifting the centering device on the bone surface, to position the center of said probe-bearing flat element on the axis of the magnet in correspondence of the maximum taken value, the coincidence of the axes of the distal hole and the magnet being determined, in case of a same reading for the three probes, while in case of three different values said coincidence of the axes is obtained by rotatiing the axis of the centering device kept still against the bone surface, until equal values are obtained for the three probes.

More particularly, said bar-shaped support of the magnet is fastened to the proximal end of the nail by coupling to the conical threading provided at the end of said nail, or alternatively it may be fastened according to different methods to the intramedullary nail.

Besides, said probe-bearing flat element is substantially shaped as a disk which is anchored coaxially and translatably to said distal centering device to allow to move as near as possible to the bone, compatibly with the operating requirements, and so as to reduce as much as possible any error possibilities.

Further characteristics and advantages of this invention shall appear more clearly from the following detailed description, made with reference to the enclosed figures, wherein:
Fig. 1 shows schematically a universal intramedullary nail of a known type, while
Fig. 2 shows a magnetic device realized according to this invention, applied inside the nail of Fig. 1, and a taking device positioned outside the bone and coaxially to the distal hole of said nail.

With reference to the above mentioned figures, the magnetic device subject matter of this invention suitable to allow the individuation of the correct location and direction of the distal hole of the nail inserted into the bone in a known way, is utilizable in the techniques of reduction of bone fractures, such as those of the femur, the tibia and the like.

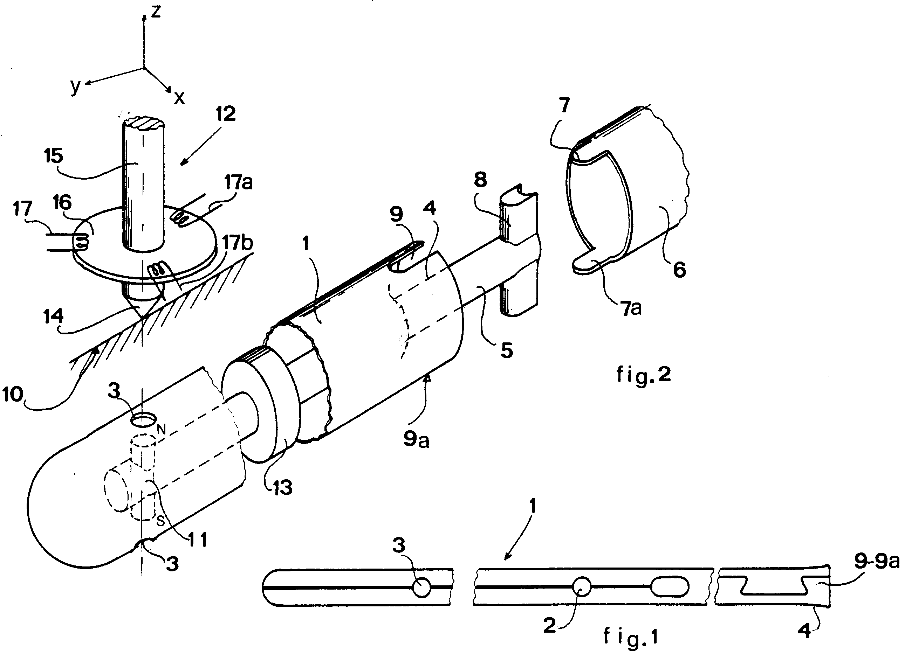

In fact, for these applications, a universal intramedullary nail is used, Fig. 1, constituted by an elongated metal body 1, axially hollow, slightly arched and provided, near the opposing ends, with a transversal hole 2 called a proximal hole, i.e. a hole placed in the nearest position relatively to the operator which drives in the nail, and a hole 3, called a distal hole.

The proximal end of nail 1 serves to house a bar-shaped bolt 5, movably anchored to an arched driving in grip 6 by embedding of two opposing teeth 7-7a protruding from said grip within a transversal small bar 8 and embedding of said small bar into two opposing indentations 9-9a provided on the proximal end of the universal nail 1. Said indentations 9-9a are the alignment means of the small bar 5 and the grip angled relatively to the nail axis.

The conical threading 4 of the proximal end of the nail allows the tightening of elements 5 and 6 on said nail, by means of a special conical bolt.

This stated, the magnetic device subject matter of this invention for the individuation of the correct location and direction of the distal hole (in order to allow the subsequent corresponding hole through bone 10) ( Fig. 2) is substantially constituted by the combination of a cylindrical permanent magnet 11 and a system for taking the intensity of the magnetic flow emitted by the magnet, which system is globally indicated by 12 on Fig. 2.

More particularly, the magnetic device subject matter of this invention is constituted, according to a preferred embodiment of practical realization, by a cylindrical magnet 11 anchored to the end of a cylindrical support 13 having a guide function within the nail cavity, which support may either be anchored to bolt 5 already provided for driving in the nail or be constituted by a bar-shaped element anchored to said conical threading 4 to which bolt 5 is also anchored.

Magnet 11, having been transversally anchored to the end of support 13, is inserted into the nail and positioned with its geometrical axis coaxial to the axis of distal hole 3 (Fig. 2). Through a suitable cut into the muscle which encloses the bone, the conical point 14 of a bar-shaped element 15 - whose external shape is similar to the one of a distal centering device of a known type - is brougth in touch with the bone surface 10. This special distal centering device 15 is brought in touch with the bone surface in a region tentatively near the distal hole and is so supported as to be caused to shift on said surface 10 and also to rotate, keeping however the conical point 14 always still in the same point of touch, for reasons that shall be explained later on.

Coaxially to said distal centering device 15 a flat disk 16 is anchored (possibly translable and stoppable at different heights on the centering device 15) on which three probes for taking the magnetic flow (for instance of the type commonly known as Hall sensor) emitted by the magnet which polarities N-S are arranged as shown on Fig. 2. Said probes, indicated by 17-17a-17b, are provided on a circumference coaxial to the axis of the centering device 15.

As the centering device is positioned on a region of the bone surface which may be obviously near the magnet axis and the distal hole without practically never coinciding with it because of the bending and length of the nail, the three probes arranged at 120° to one another will obviously indicate different values of the intensity of the magnetic field and therefore said three different values induce in the probes three different values of induced current.

To elaborate said three different values of signals emitted by the probes, a viewer-computer is utilized, according to this invention, which is so programmed as to indicate the takings of the three probes and to calculate the mean.

Then, to allow the individuation of the location and direction of the magnet axis (and therefore also of the distal hole), the distal centering device 14 is caused to shift on the bone surface in several directions and according to shiftings of different width, so as to allow the viewer to maximize the mean value of the three takings. In correspondence of the maximum mean value obtained, the center of the probe-bearing disk 16 will be located on the magnet axis.

If said maximum mean value is obtained from the takings of three equal values emitted by the probes, the magnet axis coincides with the one of the distal hole, if, on the contrary, the maximized mean is obtained from three different values obtained from the probes, to obtain the coaxiality of the axis of the magnet with that of the distal hole, it suffices to slowly rotate the axis of the distal centering device, keeping point 14 leaning still against the bone surface 10 (as indicated on Fig. 2).

In any case the taking of three equal values of magnetic intensity emitted by the probes ensures the coincidence of the axis of the centering device 14 with that of magnet 11.

Always according to this invention, to make the viewer-computer apparatus simpler, one can elaborate by means of one only viewer the variance (difference between the individual values read) of the readings of the three probes relatively to the mean value of same, obtaining in this way the coincidence of the axes of the magnet with those of the distal centering device when the value of the variance becomes null; such circumstance can be pointed out by the instrument through a sound or light signal.

Having so individuated the location and direction of the distal hole, it will be possible, upon removal of the magnet and the associated support from the nail cavity, to make in the bone cortical region a hole whose axis is perfectly aligned with the axis of the distal hole, performing the trepanation in the direction of the axis of the distal centering device.

Besides, the research of the location and direction of the magnet axis may be performed several times by means of automatized acquisition systems, all of them falling within the invention scope of the above described device.

Always according to this invention and to another embodiment, the individuation of the location and direction of the geometric axis of the magnet (and therefore also of that of the distal hole), after the driving in of the nail and the magnet positioned within said nail with its axis coinciding with that of the distal hole, may be obtained by positioning, outside the bone surface, a flat element perpendicular to the magnetization axis of the magnet; such perpendicularity may be obtained by anchoring the flat element to a support outside the bone, in a direction substantially parallel to the axis of the magnet support, before the introduction of the magnet into the nail, which external support may be caused to be integral with bolt 5 or the bar-shaped support of the magnet in the region where said support is screwed to the nail or the grip.

On said flat element a taking probe is then positioned, shifting which (on the same plane) three points can be determined having the same intensity value; having individuated said three points with the same intensity value, one can calculate through a computer the relevant isointensity line, which is a circumference. The axis of the magnet and therefore also the axis of the distal hole are individuated by the direction perpendicular to the probe-bearing disk and passing through the center of said circumference.

Obviously, to increase the measurement exactness one may take several isointensity circumferences and determine the point which is in the gravity center of the centers.

Lastly, to increase in any case the measurement exactness, rare earth magnets may be utilized, capable of emitting more intense measurable signals.

Obviously, in the practice, modifications and variants structurally and functionally equivalent may be introduced e.g. a number of probes also lower then three, in the invention as described according to same of its possible embodiments, without exceeding the protection scope of said invention.

## Claims

1. Magnetic device for the individuation of the correct location and direction of the axis of a hole, in particular of the distal hole of a universal nail driven into a bone according to the techniques of reduction of bone fractures, and constituted by an elongated and slightly arched hollow body, provided with a proximity hole, a distal hole and an inner conical threading, characterized in that it is constituted by a permanent cylindrical magnet, transversally anchored to an end of a bar-shaped element guidedly inserted into the existing cavity of the universal nail, until the geometric axis of said magnet is stably coxially positioned relatively to the axis of the distal hole (or anyhow fastened to the nail coaxially to the distal hole); by a bar-shaped cone-pointed element performing the function of distal centering device which is caused to shift until it comes in touch with the bone surface in the region of the distal hole to be individuated; by a flat element, anchored coaxially and transversally to said distal centering device and bearing three (or more) probes taking the intensity of the magnetic field emitted by said magnet and arranged at 120° to one another on a circumference; said three probes being connected to a viewer-computer so programmed as to elaborate and indicate the values of the takings of the three probes and to calculate the mean of said three values, so as to allow, by shifting the centering device on the bone surface, to position the center of said probe-bearing element on the axis of the magnet in correspondence of the maximum mean value taken, the coincidence of the axes of the distal hole and the magnet being determined, in case of readings of equal values for the three probes, while in case of three different values, said coincidence of the axes is obtained by rotating the axis of the centering device kept still against the bone surface, until equal values are obtained for the three probes.

2. Device according to claim 1, characterized in that said bar-shaped support of the magnet is fastened to the proxiaml end of the nail by coupling to the conical threading provided at the end of said nail.

3. Device according to claim 1, characterized in that said probe-bearing element is shaped as a disk or the like, coaxially anchored to said distal centering device and translable to allow several positions relatively to the bone surface.

4. Device according to one or more of the preceding claims, characterized in that it is provided, for the individuation of the location and direction of the axis of said magnet, with a flat element placed outside the bone, arranged perpendicularly to the geometric axis of said magnet and supported by an arm or the like integral with said support of the magnet and/or said arched grip, on which flat element at least a probe translable on said flat element is positioned, so as to trasmit to a viewer-computer three points having the same intensity value which define, through said computer, an isointensity line coinciding with a circumference, the magnet axis being defined and individuated by the direction perpendicular to said probe-bearing disk and passing through the center of said circumference.

5. Magnetic device according to the preceding claims, characterized in that it is realized to the purposes and for the utilizations above specified according to what has been described and illustrated.
